# EUROPÄISCHE PATENTSCHRIFT

(11) **EP 3 545 910 B1**
(45) Veröffentlichungstag und Bekanntmachung des Hinweises auf die Patenterteilung: **01.12.2021**
(21) Anmeldenummer: 19165548.9
(22) Anmeldetag: 27.03.2019
(51) Int. Cl.: A61F 2/38, A61B 5/00, A61F 2/32

(54) **IMPLANTAT ZUR DIAGNOSE UND/ODER BEHANDLUNG ENTZÜNDLICHER GEWEBEZUSTÄNDE**
IMPLANT FOR DIAGNOSIS AND/OR TREATMENT OF INFLAMMATORY TISSUE CONDITIONS
IMPLANT DE DIAGNOSTIC ET / OU DE TRAITEMENT DES ÉTATS DE TISSU INFLAMMATOIRES

(30) Priorität: 30.03.2018 DE 102018204949
(43) Veröffentlichungstag der Anmeldung: 02.10.2019
(73) Patentinhaber: Clasbrummel, Bernhard, 70597 Stuttgart (DE)
(72) Erfinder: Clasbrummel, Bernhard, 70597 Stuttgart (DE)
(74) Vertreter: Patentanwaltskanzlei Cartagena

(56) Entgegenhaltungen:
- WO-A1-97/33513
- WO-A1-2008/035089
- WO-A1-2014/209916
- US-A1- 2002 077 556

## Beschreibung

Die nachfolgend beschriebene Erfindung betrifft ein Implantat, das zur Implantation in einen menschlichen oder tierischen Körper vorgesehen ist.

Das Einsetzen von Implantaten in einen menschlichen oder tierischen Körper erfordert meist einen operativen Eingriff, der eine Wundheilung nach sich zieht. Bei der Wundheilung kann es verschiedene Komplikationen geben, die den Heilungsverlauf negativ beeinflussen können. So kann es im postoperativen Heilungsverlauf, insbesondere unmittelbar nach dem Einsetzen des Implantats, zu Entzündungen in an das Implantat angrenzendem Gewebe kommen. Derartige Entzündungen kommen in 1-2 % der Fälle vor und sind in der Regel durch Bakterien verursacht. Wenn sie nicht frühzeitig erkannt werden, können Folgeeingriffe erforderlich sein. In schweren Fällen kann es sogar erforderlich sein, das Implantat wieder zu entfernen.

Es gilt der Grundsatz, dass Entzündungen umso besser zurückgedrängt werden können, je früher sie detektiert werden. Dies ist jedoch im Fall von Implantaten schwierig. Eine visuelle Überwachung des Heilungsverlaufs ist in der Regel nicht möglich und Entzündungsmarker wie C-reaktives Protein (CRP) sowie Entzündungsparameter wie die Erhöhung der Körpertemperatur oder ein Anstieg der Leukozytenzahl sind zu unspezifisch.

Aus der WO 2014/209916 A1 und der WO 2008/035089 A1 ist es bekannt, Implantate zur Detektion entzündlicher Zustände mit Sensoren auszustatten. Bei den Sensoren kann es sich um physikalische Sensoren wie Druck- und Temperatursensoren oder um chemische oder biologische Sensoren handeln. Die Sensoren können kontaktlos angesteuert werden.

Aus der US 2002/0077556 A1 ist eine implantierbare Sonde, mit welcher der hämodynamische Blutdruck in einer Herzkammer gemessen werden kann, bekannt. Die Sonde umfasst fotoelektrische Zellen, die im Betrieb einen Fotostrom erzeugen, der in Abhängigkeit des Blutdrucks variiert wird. Die Variation kann gemessen und drahtlos an einen externen Empfänger übertragen werden.

Aus der WO 2008/035089 A1 sind Implantate bekannt, die ein antimikrobielles Mittel umfassen, welches gezielt aktiviert werden kann. Das Implantat kann zu diesem Zweck eine Kommunikationseinheit umfassen, über welche die Freigabe des antimikrobiellen Mittels gesteuert werden kann.

Die WO 2014/209916 A1 befasst sich mit dem postoperativen Monitoring eines künstlichen Kniegelenks. Im Fokus steht hierbei eine Überwachung des Implantats, die sich über mehrere Jahre hinzieht und u.a. darauf abzielt, Abnutzungserscheinungen zu erfassen. Hierzu werden diverse Sensoren an und in den Komponenten des Kniegelenks angeordnet. Hierbei kann es sich um Drucksensoren, Kontaktsensoren, Positionssensoren, chemische Mikrosensoren, Gewebestoffwechselsensoren, mechanische Belastungssensoren und Temperatursensoren handeln. Angesteuert werden diese Sensoren bevorzugt über eine drahtlose Verbindung. Es ist vorgesehen, einen Sensor drahtlos zu aktivieren, mit ausreichend elektrischer Energie zu versorgen, um Daten zu erfassen und diese Daten anschließend an einen externen Empfänger zu übermitteln.

Der vorliegenden Erfindung lag die Aufgabe zugrunde, nach dem Einsetzen eines Implantats entzündliche Gewebezustände in an das Implantat angrenzendem Gewebe und in Körperflüssigkeiten, beispielsweise Gelenkflüssigkeit oder Knochengewebe, möglichst frühzeitig zu detektieren.

Zur Lösung dieser Aufgabe schlägt die Erfindung das Implantat mit den Merkmalen des Anspruch 1 vor. Weiterbildungen der Erfindung sind Gegenstand von Unteransprüchen.

Das erfindungsgemäße Implantat ist zur Implantation in einen menschlichen oder tierischen Körper vorgesehen. Es zeichnet sich durch die folgenden Merkmale aus:
a. es umfasst eine Sensorik zur Erfassung elektromagnetischer Wellen, die von einer Umgebung des Implantats emittiert oder reflektiert werden, und
b. es umfasst eine Datenübertragungseinheit, die von der Sensorik gelieferte Daten kontaktlos an einen Empfänger übertragen kann.

Erfindungsgemäß umfasst die Sensorik zur Erfassung der elektromagnetischen Wellen ein elektronisches Bauteil, das für die Detektion elektromagnetischer Wellen im sichtbaren Bereich des elektromagnetischen Spektrums uausgelegt ist, und/oder die Sensorik ist dazu ausgebildet, elektromagnetische Wellen im Bereich von 200 nm bis 700 nm zu empfangen.

Die Sensorik ist derart ausgelegt, dass sie direkt oder indirekt mögliche Änderungen von Eigenschaften von an das Implantat angrenzendem Körpergewebe oder in Körperflüssigkeiten wie der erwähnten Gelenkflüssigkeit, insbesondere die Bildung entzündlicher Gewebezustände, erfassen kann. Insbesondere ist die Sensorik auch dazu ausgelegt, dass sie Veränderungen an oder auf Oberflächen des Implantats und in der unmittelbar an das Implantat angrenzenden Umgebung erfassen kann. Bei einer bakteriellen Entzündung lagern sich auf den Oberflächen des Implantats sehr schnell Biofilme ab. Diese umfassen in der Regel eine Schleimschicht (Film), in die Mikroorganismen eingebettet sind. Derartige Biofilme lassen sich mit der Sensorik problemlos detektieren. Die Anordnung der Sensorik innerhalb des Implantats ermöglicht - falls erforderlich - sogar eine Live-Überwachung des Heilungsverlaufs. Bildet sich eine Entzündung, so kann diese noch im Entstehen detektiert werden. So werden frühzeitige Gegenmaßnahmen ermöglicht.

Bei der Datenübertragungseinheit handelt es sich bevorzugt um ein miniaturisiertes Gerät, das Daten mit Hilfe modulierter elektromagnetischer Wellen im Radiofrequenzbereich drahtlos übertragen kann. So kann es sich bei dem Gerät beispielsweise um einen Bluetooth-, WLAN-, Zigbee-, GSM-, CDMA-, UMTS- oder LTE-Chip handeln.

Der Empfänger ist in der Regel ein Datenverarbeitungsgerät wie ein Computer, das über einen zur Datenübertragungseinheit korrespondierenden Empfangschip verfügt.

Das Implantat kann ein- oder mehrteilig ausgebildet sein. Bei einem mehrteiligen Implantat kann es bevorzugt sein, dass eine Sensorik und eine Datenübertragungseinheit in jedes der Teile integriert sind.

Es ist bevorzugt, dass die Sensorik bei einem mehrteiligen Implantat dazu ausgebildet und/oder innerhalb einem der Implantat-Teile derart angeordnet ist, dass insbesondere Grenzflächen zwischen den einzelnen Implantat-Teilen beobachtet werden können.

Das Implantat weist in einer besonders bevorzugten Ausführungsform die folgenden zusätzlichen Merkmale auf:
a. Es umfasst mindestens einen Hohlraum, in dem die Sensorik und die Datenübertragungseinheit angeordnet sind, und
b. es ist derart ausgebildet, dass die elektromagnetischen Wellen die in dem Hohlraum angeordnete Sensorik erreichen können.

Es ist zweckmäßig, die Sensorik und die Datenübertragungseinheit in einem oder mehreren Hohlräumen innerhalb des Implantats anzuordnen. Dabei ist es durchaus möglich, dass ein Bauteil der Sensorik oder der Datenübertragungseinheit den Hohlraum nach außen hin begrenzt. Weiterhin ist es durchaus möglich, dass die Sensorik und die Datenübertragungseinheit in unterschiedlichen Hohlräumen innerhalb des Implantats angeordnetsind.

Es ist in bevorzugten Ausführungsformen nicht vorgesehen, die Sensorik und die Datenübertragungseinheit nach erfolgter Heilung in Folge einer Implantation aus dem Implantat zu entfernen. Vielmehr sollen sie in der Regel dauerhaft in dem Implantat verbleiben. Aus diesem Grund ist es bevorzugt, dass der oder die Hohlräume nach außen hin bevorzugt hermetisch versiegelt sind.

Ungeachtet dessen ist es in diesem Fall erforderlich, dass die Sensorik die zu erfassenden elektromagnetischen Wellen noch empfangen kann. Es sollten also bevorzugt zumindest die Teile des Implantats, die sich zwischen der Sensorik und dem umliegenden Gewebe befinden, von elektromagnetischen Wellen durchdrungen werden können. Das Implantat muss also mindestens teilweise aus einem für die elektromagnetischen Wellen transparenten Material bestehen.

In einer weiteren, besonders bevorzugten Ausführungsform weist das Implantat mindestens eines der folgenden zusätzlichen Merkmale auf:
a. Es besteht mindestens teilweise aus Glas, insbesondere aus durchsichtigem Glas.
b. Es besteht mindestens teilweise aus einer Glaskeramik.
c. Es besteht mindestens teilweise aus einer Keramik.
d. Es besteht mindestens teilweise aus einem Kunststoff, insbesondere aus einem Polyolefin wie Polyethylen.

Diese Werkstoffe sind in aller Regel für Wellen weiter Bereiche des elektromagnetischen Spektrums transparent und genügen daher den obigen Anforderungen. In vielen Fällen ist allerdings insbesondere die Verwendung von Kunststoffen als für die elektromagnetischen Wellen transparentes Material besonders bevorzugt.

Als Glas ist der Einsatz sogenannten Bioglases denkbar, welches üblicherweise eine hohe Verträglichkeit mit körpereigenem Gewebe aufweist.

Glaskeramiken sind Werkstoffe, die aus Glasschmelzen durch gesteuerte Kristallisation hergestellt werden. Sie zeichnen sich in aller Regel durch eine hohe Durchlässigkeit für Infrarotstrahlung aus.

Die Verwendung keramischer Komponenten in Implantaten ist Stand der Technik.

Als Kunststoffe sind neben den bereits genannten Polyolefinen insbesondere solche mit einem hohen Verschleißwiderstand und einer hohen Verträglichkeit zu körpereigenem Gewebe bevorzugt. In Frage kommen insbesondere fluorierte Polymere wie Teflon und Polyetherketone wie Polyetheretherketon.

Besonders bevorzugt umfasst das Implantat eine metallische Stützstruktur, beispielsweise aus Titan oder einem Edelstahl, die bereichsweise oder vollständig von dem Glas, der Glaskeramik oder dem Kunststoff eingeschlossen wird.

In vielen Fällen ist es bevorzugt, dass das Implantat metallische Komponenten, Komponenten aus dem Kunststoff und Komponenten aus der Glaskeramik oder gläserne Komponenten umfasst.

Es ist bevorzugt, dass sich das Implantat durch mindestens eines der folgenden zusätzlichen Merkmale auszeichnet:
a. Das mindestens eine von der Sensorik umfasste elektronische Bauteil umfasst einen Photowiderstand, eine Photodiode, einen Phototransistor oder ein anderes lichtempfindliches elektronisches Bauelement.
b. Das elektronische Bauteil ist ein lichtempfindlicher elektronischer Bildsensor zur Erfassung der elektromagnetischen Wellen.
c. Der mindestens eine lichtempfindliche elektronische Bildsensor ist ein CCD-Sensor oder ein CMOS-Sensor.

Viele Bakterien geben bei entsprechender Lichtanregung eine Fluoreszenzantwort innerhalb des Bereichs von 200 nm bis 700 nm. Daher ist es alternativ bevorzugt, wenn die Sensorik zum Empfang von Wellenlängen in diesem Bereich ausgebildet ist.

Gemäß den unmittelbar vorstehenden Merkmalen b. und c. ist die Sensorik dazu ausgebildet, ein Bild, insbesondere ein zweidimensionales Bild, zu generieren. Das elektronische Bauteil ist also bevorzugt ein elektronischer Bildwandler. Wenn das elektronische Bauteil zum Empfang elektromagnetischer Strahlung aus dem sichtbaren Bereich ausgelegt ist, kann es ein fotografisches Abbild generieren. Damit ist es möglich, einen Heilungsverlauf auch visuell zu überwachen.

In bevorzugten Ausführungsformen ist das elektronische Bauteil dazu ausgebildet, 2D- und/oder 3D-Bilder zu generieren.

Wenn das Bauteil zum Empfang elektromagnetischer Strahlung aus dem sichtbaren Bereich ausgebildet ist, so kann die Sensorik eine oder mehrere Linsen umfassen, um einfallendes Licht fokussieren zu können.

Die Aufnahme eines Bildes ist, wie im Zusammenhang mit dem erfindungsgemäßen Verfahren erläutert werden wird, nicht immer erforderlich oder zweckmäßig. Vielmehr kann es zur Detektion entzündlicher Gewebezustände auch ausreichend sein, Wellen mit ausschließlich einer Wellenlänge (und nicht einen gesamten Bereich des elektromagnetischen Spektrums) zu erfassen und auf Intensitätsänderungen hin zu überwachen. Hierzu ist es ausreichend, wenn die Sensorik gemäß den unmittelbar vorstehenden Merkmalen a. und b. ausgebildet ist.

Es ist weiterhin bevorzugt, dass sich das Implantat durch mindestens eines der folgenden zusätzlichen Merkmale auszeichnet:
a. Es umfasst mindestens eine Steuereinheit, die die Funktionen der Sensorik und/oder der Datenübertragungseinheit steuert.
b. Die Steuereinheit ist dazu ausgebildet, eine unmittelbare Weiterleitung der von der Sensorik gelieferten Daten mittels der Datenübertragungseinheit an einen Empfänger zu veranlassen.
c. Die Steuereinheit ist dazu ausgebildet, die von der Sensorik gelieferten Daten zu speichern und zu einem späteren Zeitpunkt an die Datenübertragungseinheit zu übertragen.
d. Die Steuereinheit ist dazu ausgebildet, die von der Sensorik gelieferten Daten zu bearbeiten, beispielsweise zu komprimieren, oder mit zuvor erfassten Daten oder gespeicherten Referenzdaten zu vergleichen und/oder zu kombinieren.
e. Die Steuereinheit ist in dem Hohlraum mit der Sensorik oder in einem weiteren Hohlraum innerhalb des Implantats angeordnet.

Bei der Steuereinheit handelt es sich im einfachsten Fall um einen integrierten Schaltkreis (IC). Gegebenenfalls ist die Steuereinheit mit der Sensorik und/oder der Datenübertragungseinheit zu einem gemeinsamen integrierten Schaltkreis zusammengefasst.

Bevorzugt wird die Sensorik über die Steuereinheit aktiviert und deaktiviert. Dies kann auf ein externes Signal hin erfolgen, es ist aber auch möglich, dass die Steuereinheit selbsttätig die Sensorik in regelmäßigen Abständen aktiviert und die erfassten Daten bis zu ihrem späteren Abruf in einem Datenzwischenspeicher ablegt.

Die Komprimierung der der Sensorik gelieferten Daten kann insbesondere vor ihrer Übertragung durch die Datenübertragungseinheit zweckmäßig sein.

In einer besonderen Ausführungsform kann die Steuereinheit auch eine erste Auswertung der gelieferten Daten übernehmen und beispielsweise lediglich bei einer Veränderung oder einer erkannten Entzündung ein Warnsignal mittels der Datenübertragungseinheit an den Empfänger schicken.

In vielen Ausführungsformen zeichnet sich das Implantat durch mindestens eines der folgenden zusätzlichen Merkmale aus:
a. Es umfasst eine Lichtquelle, die Licht im für das menschliche Auge sichtbaren Bereich des elektromagnetischen Spektrums emittieren kann.
b. Es umfasst eine Strahlungsquelle, die elektromagnetische Strahlung außerhalb des sichtbaren Bereichs, insbesondere IR-Strahlung oder UV-Strahlung, emittieren kann.
   c. die Licht- und/oder die Strahlungsquelle ist mit der Steuereinheit gekoppelt, die ihre Funktionen kontrolliert.
   d. Die Licht- und/oder die Strahlungsquelle ist in dem Hohlraum mit der Sensorik, dem Hohlraum mit der Steuereinheit oder in einem weiteren Hohlraum innerhalb des Implantats angeordnet.

Bei der Lichtquelle gemäß Merkmal a. handelt es sich bevorzugt um eine übliche Licht- oder Blitzlichtquelle. Als Leuchtmittel werden bevorzugt LED-Leuchtmittel verwendet. Die Lichtquelle kommt insbesondere in Kombination mit einem der beschriebenen elektronischen Bildwandler zum Einsatz und dient dazu, die Bedingungen zur Generierung des fotografischen Abbilds zu verbessern. Das von der Lichtquelle abgegebene Licht kann durch die unmittelbare Umgebung des Implantats teilweise reflektiert werden, z.B. durch an das Implantat angrenzendes Gewebe. Das reflektierte Licht kann vom Sensor erfasst werden.

Bei der Strahlungsquelle gemäß Merkmal b. kann es sich beispielsweise um eine Infrarot-LED oder eine UV-LED handeln. Diese Strahlungsquellen können beispielweise im Rahmen des unten beschriebenen Verfahrens zum Nachweis eines Markers oder zur Desinfektion dienen.

In einigen besonders bevorzugten Ausführungsformen ist die Strahlungsquelle gemäß Merkmal b. dazu ausgebildet, Strahlung im Frequenzbereich von 10 nm bis 400 nm, besonders bevorzugt von 100 nm bis 350 nm, zu emittieren. Viele Bakterien geben bei einer Anregung mit elektromagnetischer Strahlung in diesem Frequenzbereich eine Fluoreszenzantwort innerhalb des oben genannten Frequenzspektrums. Insbesondere Strahlung am kurzwelligen Ende des Spektrums ist dazu geeignet, Bakterien abzutöten, dient also der erwähnten Desinfektion.

In möglichen Weiterbildungen des Implantats zeichnet es sich durch mindestens eines der folgenden zusätzlichen Merkmale aus:
a. Es umfasst mindestens eine Energiequelle, die die Sensorik und die Datenübertragungseinheit sowie gegebenenfalls die Steuereinheit und/oder die Licht- und/oder die Strahlungsquelle mit elektrischer Energie versorgt.
b. Die mindestens eine Energiequelle umfasst eine elektrochemische Zelle.
c. Die mindestens eine Energiequelle umfasst einen Kondensator.
d. Die mindestens eine Energiequelle umfasst eine Induktionsspule/Kopplungsspule.
e. Die mindestens eine Energiequelle umfasst einen Energiewandler.
f. Die Energiequelle ist in dem Hohlraum mit der Sensorik, dem Hohlraum mit der Steuereinheit, dem Hohlraum mit der Licht- und/oder die Strahlungsquelle oder in einem weiteren Hohlraum innerhalb des Implantats angeordnet.

In der Ausführungsform als elektrochemische Zelle ist die Energiequelle bevorzugt derart ausgebildet, dass sie die Sensorik und die damit gekoppelten Komponenten des Implantats für mindestens 24 Stunden mit elektrischer Energie versorgen können. Dadurch ist gewährleistet, dass ein Heilungsverlauf zumindest unmittelbar nach einer Implantation unterbrechungsfrei überwacht werden kann.

Der Kondensator kann insbesondere in Kombination mit der vorstehend beschriebenen Lichtquelle benötigt werden, nämlich insbesondere wenn es sich bei dieser um eine Blitzlichtquelle handelt.

Wenn die Energiequelle als Induktionsspule/Kopplungsspule ausgebildet ist, kann Energie kontaktlos von außerhalb des Implantats übertragen werden. Besonders bevorzugt umfasst das Implantat eine Kombination aus der Induktionsspule/Kopplungsspule und der elektrochemischen Zelle, wobei letztere in diesem Fall bevorzugt wieder aufladbar ausgebildet ist.

Als Energiewandler wird im Kontext dieser Anmeldung ein Bauteil verstanden, welches die Gewinnung elektrischer Energie aus mechanischer Energie und/oder Wärmenergie ermöglicht. Der Energiewandler kann elektrische Energie aus der Umgebungstemperatur, aus Vibrationen, aus Bewegung und aus Druck gewonnen werden. Beispielsweise können als Energiewandler Bauteile verwendet werden, bei denen durch einen piezoelektrischen Effekt mechanischer Druck in elektrische Spannung umgewandelt wird.

In einigen bevorzugten Ausführungsformen umfasst das Implantat eine Kombination aus dem Energiewandler und der elektrochemischen Zelle, wobei letztere auch in diesem Fall bevorzugt wieder aufladbar ausgebildet ist.

Es ist bevorzugt, dass sich das Implantat durch das folgende zusätzliche Merkmal auszeichnet:
a. Die Induktionsspule/Kopplungsspule ist Bestandteil der Datenübertragungseinheit.

In einer bevorzugten Ausführungsform ist die Induktionsspule für eine induktive Datenübertragung ausgelegt. Hierdurch können Daten sowie die benötigte Energie gemeinsam und kontaktlos übertragen werden. Derartige Kopplungen von Energieversorgung und Datenübertragung sind bereits vielerorts im Einsatz, besonders sind in diesem Zusammenhang beispielsweise Cochlea-Implantate und die RFID-Technologie bekannt.

Es ist weiterhin bevorzugt, dass sich das Implantat durch mindestens eines der folgenden zusätzlichen Merkmale auszeichnet:
a. Es umfasst eine Diagnosesubstanz zum Nachweis von Entzündungen.
b. Die Diagnosesubstanz ist auf eine Außenseite des Implantats aufgebracht.
c. Es umfasst ein Depot mit der Diagnosesubstanz.

Die Diagnosesubstanz umfasst bevorzugt ein Koppelmolekül, das an eine als Folge einer Entzündung im Gewebe gebildeten molekularen Struktur - worunter einzelne Moleküle, Molekülverbände oder molekulare Oberflächenstrukturen zu verstehen sind - spezifisch ankoppelt bzw. bindet. Die Koppelmoleküle sind bevorzugt mit einem biochemischen Marker versehen, so dass eine Anreicherung von Koppelmolekülen in entzündetem Gewebe oder in einer Körperflüssigkeit oder an einer Oberfläche des Implantats feststellbar ist.

Vorzugsweise werden Marker verwendet, die elektromagnetische Wellen, etwa sichtbares Licht oder Röntgenstrahlen absorbieren und beispielsweise anhand ihres Absorptionsspektrums detektiert werden können. Besonders bevorzugt sind Marker-Farbstoffe, die im nahen Infrarot (NIR) absorbieren. Besonders bevorzugt sind Fluoreszenzfarbstoffe, da diese in der Regel in einem von ihrem Fluoreszenzspektrum verschiedenen Wellenlängenbereich absorbieren, so dass das von ihnen ausgesendete Fluoreszenzlicht leicht aus dem Anregungslicht herausgefiltert werden kann.

Zu Diagnosezwecken kann - beispielsweise auf ein Signal der Steuereinheit hin - die Diagnosesubstanz aus dem Depot freigesetzt werden. Im Falle einer Entzündung wird sie sich am Ort der Entzündung anreichern. Bei Anregung mit einer elektromagnetischen Welle wird ein als Marker verwendeter Fluoreszenzfarbstoff elektromagnetische Wellen einer längeren Wellenlänge emittieren, die beispielsweise von der Sensorik des Implantats detektiert werden können.

Es ist weiterhin bevorzugt, dass sich das Implantat durch mindestens eines der folgenden zusätzlichen Merkmale auszeichnet:
a. Es umfasst einen Wirkstoff zur Behandlung von Entzündungen.
b. Der Wirkstoff ist auf eine Außenseite des Implantats aufgebracht.
c. Es umfasst ein Depot mit dem Wirkstoff.

Das Vorhandensein des Wirkstoffs ermöglicht neben einer Diagnose von Entzündungen auch deren unmittelbare Behandlung. So kann beispielsweise das Depot mit der Steuereinheit verbunden sein und auf ein Signal der Steuereinheit hin den Wirkstoff freisetzen.

Das Implantat zeichnet sich durch mindestens eines der folgenden zusätzlichen Merkmale aus:
a. Das Implantat umfasst ein künstliches Gelenk.
b. Das Implantat ist ein Herzschrittmacher.
c. Das Implantat ist ein implantierbares Hörgerät.

Das künstliche Gelenk ist bevorzugt ausgewählt aus der Gruppe mit Schultergelenk, Ellenbogengelenk, Handgelenk, Hüftgelenk, Kniegelenk und Sprunggelenk.

Bevorzugt handelt es sich bei dem Implantat also um eine Endoprothese, die für einen dauerhaften Verbleib im Körper vorgesehen ist und ein geschädigtes Gelenk ganz oder teilweise ersetzt.

Ganz besonders bevorzugt ist das Implantat ein künstliches Hüftgelenk. Dieses umfasst in der Regel einen Hüftschaft, ein daran fixiertes, als Kugelkopf ausgebildetes Kopfteil und eine Hüftpfanne, in der sich der Kugelkopf bewegen kann. Der Hüftschaft besteht bevorzugt aus einem Metall wie Titan, der Kugelkopf erfindungsgemäß bevorzugt aus der Keramik, dem Glas oder der Glaskeramik. Die Hüftpfanne besteht bevorzugt aus Polyethylen mit einer metallischen Stützstruktur, beispielsweise aus Titan, oder gleichfalls einem Metall wie Titan. Insbesondere im letzteren Fall ist dann zwischen dem Kopfteil und der Hüftpfanne ein Inlay aus Kunststoff, insbesondere dem erwähnten Polyethylen, oder einer Keramik, angeordnet. Die Sensorik des Implantats ist bevorzugt innerhalb des Kopfteils angeordnet.

In aller Regel ist die Sensorik bevorzugt in Bereichen des Implantats angeordnet, die in unmittelbarer Nachbarschaft der Gelenkverbindungen liegen oder in Bereichen, die an oder in vorhandene Knochen angebunden werden sollen. Entzündungen wie eine bakterielle Infektion der Gelenkhöhle oder eine Osteitis sind schwer zu diagnostizieren, eine frühzeitige Diagnose kann in vielen Fällen den Implantaterhalt sichern.

Das erfindungsgemäße Implantat ermöglicht die Diagnose und/oder Behandlung entzündlicher Gewebezustände. Ein entsprechendes Verfahren umfasst stets die folgenden Schritte a. und b.:
a. Mittels der Sensorik des oben beschriebenen Implantats werden elektromagnetische Wellen erfasst, deren Eigenschaften von der Umgebung des Implantats abhängig sind, insbesondere vom Zustand eines an das Implantat angrenzenden Gewebes.
b. Von der Sensorik gelieferte Daten werden mittels der Datenübertragungseinheit kontaktlos an den Empfänger übertragen.

Vor den Schritten a. und b. ist das Implantat natürlich in einen menschlichen oder tierischen Körper zu implantieren.

Das dem Verfahren zugrunde liegende Prinzip wurde bereits bei der Beschreibung des erfindungsgemäßen Implantats erläutert. Entsprechend ist klar, dass es sich bei dem zu detektierenden Zustand des an das Implantat angrenzenden Gewebes insbesondere um einen Entzündungszustand handelt.

Es gibt zwei besonders bevorzugte Varianten des Verfahrens:
In einer ersten besonders bevorzugten Ausführungsform des Verfahrens umfasst dieses die folgenden zusätzlichen Schritte:
a. Die Umgebung des Implantats, insbesondere das an das Implantat angrenzende Gewebe und/oder eine mit dem Implantat in Kontakt stehende Körperflüssigkeit, wird elektromagnetischer Strahlung, insbesondere im sichtbaren Bereich des elektromagnetischen Spektrums und/oder im Infrarotbereich, ausgesetzt.
b. Die elektromagnetische Strahlung wird von einer Licht- und/oder Strahlungsquelle innerhalb des Implantats oder außerhalb des menschlichen oder tierischen Körpers, insbesondere einer der oben beschriebenen Licht- und/oder Strahlungsquellen, emittiert.
c. Von der Umgebung, insbesondere dem Gewebe und/oder der Körperflüssigkeit, emittierte oder reflektierte elektromagnetische Wellen werden mittels mindestens eines lichtempfindlichen elektronischen Bildsensors erfasst und in ein Bild umgewandelt, das mittels der Datenübertragungseinheit an den Empfänger übertragen wird.

In dieser Variante erfolgt eine Diagnose der Umgebung, indem ein fotografisches Abbild oder ein Wärmebild der Umgebung, beispielsweise des Gewebes oder der Körperflüssigkeit, generiert wird.

In einer zweiten besonders bevorzugten Ausführungsform des Verfahrens umfasst dieses die folgenden zusätzlichen Schritte:
a. Die Umgebung des Implantats, insbesondere das an das Implantat angrenzende Gewebe und/oder eine mit dem Implantat in Kontakt stehende Körperflüssigkeit, wird mit einem biochemischen Marker zum Nachweis von Entzündungen in Kontakt gebracht.
b. Die Umgebung des Implantats, insbesondere das an das Implantat angrenzende Gewebe und/oder die Körperflüssigkeit, wird elektromagnetischer Strahlung ausgesetzt, die mit einem Absorptions- und/oder Fluoreszenzspektrum des Markers überlappt.
c. Die elektromagnetische Strahlung wird von einer Licht- und/oder Strahlungsquelle innerhalb des Implantats oder außerhalb des menschlichen oder tierischen Körpers emittiert.

Gemäß dieser Ausführungsform ist die Aufnahme eines Bildes nicht erforderlich. Zur Detektion entzündlicher Gewebezustände ist es ausreichend, die elektromagnetischen Wellen zu erfassen, die von dem Marker emittiert werden oder die Intensität von elektromagnetischen Wellen zu verfolgen, die von dem Marker absorbiert werden. Der Marker und seine Funktion wurden bereits ausführlich erläutert. Bevorzugt ist er Bestandteil der oben beschriebenen Diagnosesubstanz.

Weitere Merkmale, Einzelheiten und Vorzüge der Erfindung ergeben sich aus den Ansprüchen und der Zusammenfassung, deren beider Wortlaut durch Bezugnahme zum Inhalt der Beschreibung gemacht wird, der folgenden Beschreibung bevorzugter Ausführungsformen der Erfindung sowie anhand der Zeichnungen. Hierbei zeigt schematisch:
Figur 1 (geschnittene Darstellung) eine als Endoprothese ausgebildete Ausführungsform eines erfindungsgemäßen Implantats für den Ersatz eines menschlichen Oberarmkopfes, und
Figur 2 (geschnittene Darstellung) einen Hohlraum im Knochenfixationsschaft des Implantats aus Fig. 1, in dem neben einer Sensorik eine Datenübertragungseinheit und eine Lichtquelle angeordnet sind, und
Figur 3 (geschnittene Darstellung) eine als künstliches Hüftgelenk ausgebildete Ausführungsform eines erfindungsgemäßen Implantats, und
Figur 4 (Draufsicht schräg von vorne) eine als künstliches Kniegelenk ausgebildete Ausführungsform eines erfindungsgemäßen Implantats, und
Figur 5 einen Schnitt durch eine Inlay-Komponente des in Figur 4 dargestellten Kniegelenks.

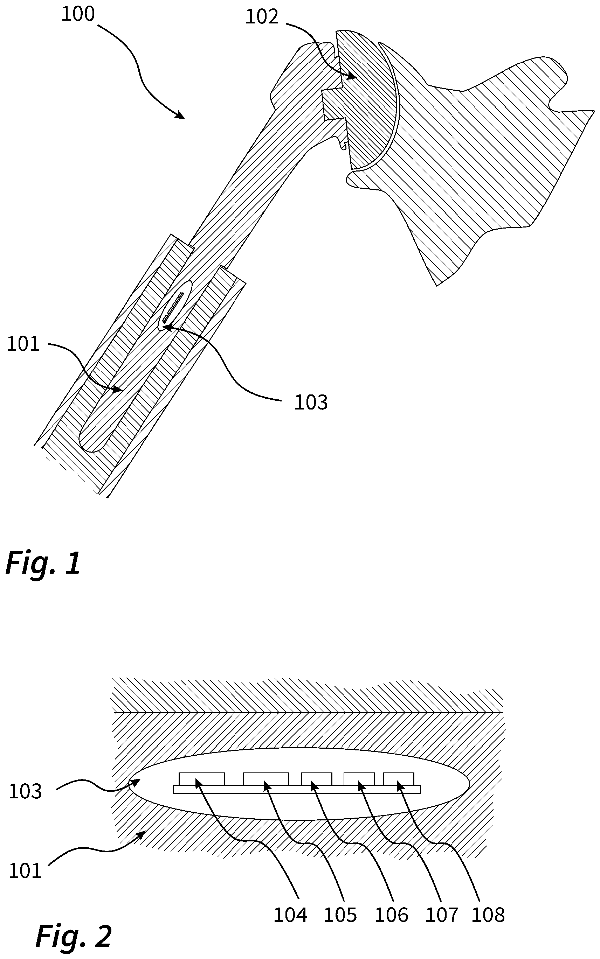

Das in Fig. 1 dargestellte Implantat 100 umfasst einen modularen Grundkörper mit einem Knochenfixationsschaft 101, der in der Abbildung im Markraum eines resezierten Oberarmknochens angeordnet ist, sowie einem Kopfteil 102, welches an eine Schultergelenkspfanne an einem Schulterblatt angelegt ist. Der Übersicht halber sind lediglich für die vorliegende Erfindung relevante Komponenten des Implantats dargestellt, auf die Darstellung beispielsweise funktionaler Teilelemente wie Weichteilfixierungsmechanismen, beispielsweise für Streck- und Abduktorweichgewebe, wurde verzichtet. Der Knochenfixationsschaft 101 besteht in Teilen aus Glas und enthält die in Fig. 2 dargestellte Sensorik.

Wie in Fig. 2 dargestellt, weist das Implantat 100 einen Hohlraum 103 innerhalb des Knochenfixationsschaftes 101 auf. In diesem sind als Sensorik 104 ein elektronischer Bildsensor, eine Lichtquelle 105, als Steuereinheit 106 ein IC, eine Datenübertragungseinheit 107 und eine Batterie 108 auf einer Platine 109 angeordnet. Die Batterie 108 kann über eine integrierte Induktionsspule (nicht dargestellt) geladen werden. Der elektronische Bildsensor 104, die Lichtquelle 105 und die Datenübertragungseinheit 107 sind jeweils mit dem IC 106 gekoppelt, der ihre Funktionen steuern kann.

Der elektronische Bildsensor 104 ist dazu ausgebildet, ein zwei- oder dreidimensionales Bild des umliegenden Gewebes zu generieren. Mittels der Lichtquelle 105 können die Lichtverhältnisse bei der Bildaufnahme eingestellt werden. Die von dem Bildsensor 104 gelieferten Daten werden mittels der Datenübertragungseinheit 107 an einen Empfänger mittels eines Bluetooth-Protokolls übertragen.

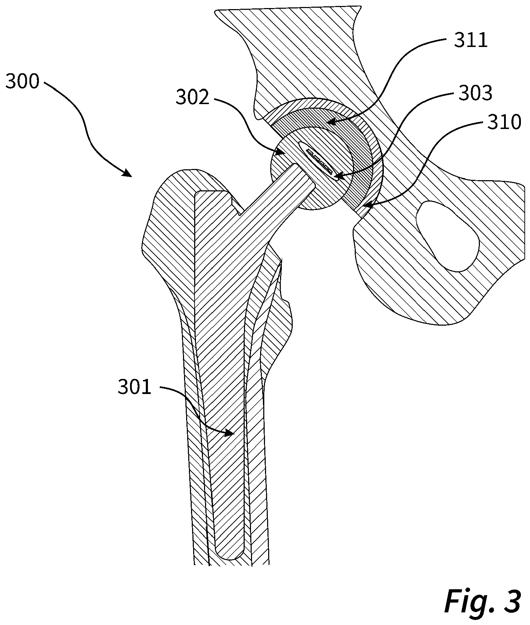

Das in Fig. 3 dargestellte Implantat 300 umfasst einen modularen Grundkörper mit einem Hüftschaft 301 aus Titan, der in der Abbildung in einem Oberschenkelknochen fixiert ist, sowie einem Kopfteil 302, welches als Kugelkopf ausgebildet ist und an eine Hüftpfanne 310 angelegt ist. Das Kopfteil 302 besteht im Wesentlichen aus einem durchsichtigen Glaskörper, in dem ein Hohlraum 303 angeordnet ist, in dem die gleiche Sensorik und die gleichen elektronischen Komponenten wie im Hohlraum 103 des Implantats 100 angeordnet sind. Auch die Hüftpfanne 310 besteht bevorzugt aus Titan. Zwischen die Hüftpfanne 310 und das Kopfteil 302 ist ein Inlay 311 aus ultrahochmolekularem Polyethylen eingefügt, das als Gleitfläche dient.

Mittels des in dem Hohlraum 303 angeordneten Bildsensors können fotografische Einzelaufnahmen, gegebenenfalls sogar Filmaufnahmen der Oberflächen des Kopfteils 302 und des Inlays 311 sowie der Grenzflächen zwischen dem Kopfteil 302 und dem Inlay 311 erzeugt werden. So lässt sich beispielsweise die Bildung eines Biofilms auf einer dieser Oberflächen oder Grenzflächen in Folge einer Entzündung nachweisen.

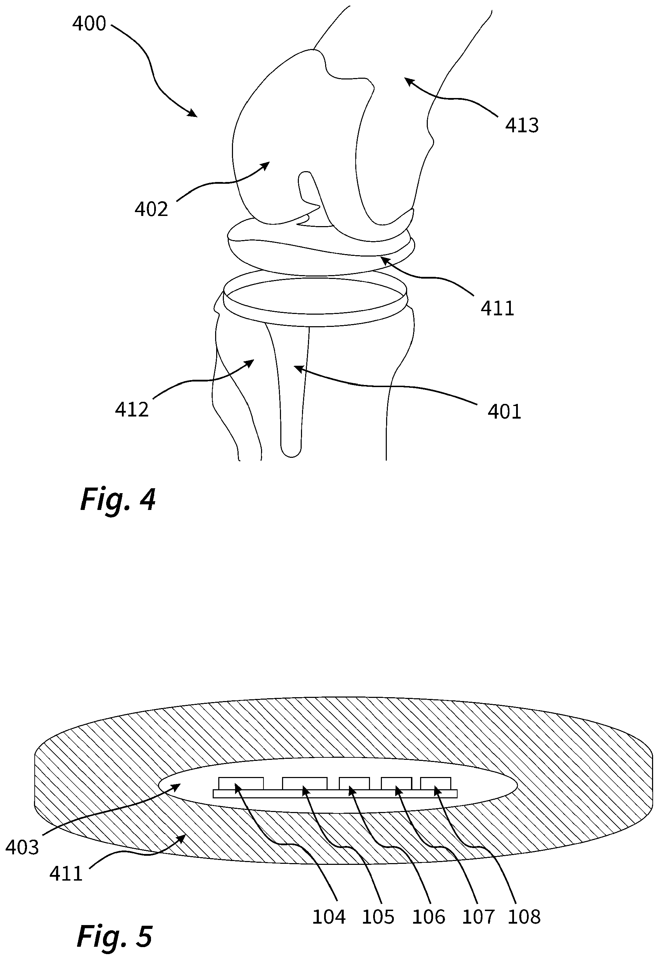

Das in Fig. 4 dargestellte Implantat 400 umfasst die in dem Schienbeinknochen 412 fixierte Tibiakomponente 401, die in dem Oberschenkelknochen 413 fixierte Femurkomponente 402 sowie das Inlay 411, auf dem die Femurkomponente 402 aufliegt. Während die Tibiakomponente 401 und die Femurkomponente 402 jeweils aus einer Cobalt-Chrom-Legierung bestehen, ist das Inlay 411 aus ultrahochmolekularem Polyethylen gefertigt.

Aus dem in Fig. 5 dargestellten Schnitt durch das Inlay 411 geht hervor, dass das Inlay 411 einen Hohlraum 403 aufweist. In diesem sind die gleiche Sensorik und die gleichen elektronischen Komponenten wie im Hohlraum 103 des Implantats 100 angeordnet.

## Patentansprüche

1. Implantat (100; 300; 400) aus der Gruppe mit Implantat, das ein künstliches Gelenk umfasst, Herzschrittmacher und implantierbares Hörgerät, mit den folgenden Merkmalen:
a. es umfasst eine Sensorik (104) zur Erfassung elektromagnetischer Wellen, die von einer Umgebung des Implantats emittiert oder reflektiert werden, und
b. es umfasst eine Datenübertragungseinheit (107), die von der Sensorik (104) gelieferte Daten kontaktlos an einen Empfänger übertragen kann,
wobei die Sensorik zur Erfassung der elektromagnetischen Wellen ein elektronisches Bauteil umfasst, das für die Detektion elektromagnetischer Wellen im sichtbaren Bereich des elektromagnetischen Spektrums ausgelegt ist, und/oder die Sensorik dazu ausgebildet ist, elektromagnetische Wellen im Bereich von 200 nm bis 700 nm zu empfangen.

2. Implantat nach Anspruch 1 mit den folgenden zusätzlichen Merkmalen:
a. Es umfasst mindestens einen Hohlraum (103; 303; 403), in dem die Sensorik (104) und die Datenübertragungseinheit (107) angeordnet sind, und
b. es ist derart ausgebildet, dass die elektromagnetischen Wellen die in dem Hohlraum (103) angeordnete Sensorik (104) erreichen können.

3. Implantat nach Anspruch 1 mit einem der folgenden zusätzlichen Merkmale:
a. Es besteht mindestens teilweise aus Glas.
b. Es besteht mindestens teilweise aus einer Glaskeramik.
c. Es besteht mindestens teilweise aus einer Keramik.
d. Es besteht mindestens teilweise aus einem Kunststoff, insbesondere einem Polyolefin wie Polyethylen.

4. Implantat nach einem der vorhergehenden Ansprüche mit mindestens einem der folgenden zusätzlichen Merkmale:
a. Das mindestens eine von der Sensorik (104) umfasste elektronische Bauteil umfasst einen Photowiderstand, eine Photodiode, einen Phototransistor oder ein anderes lichtempfindliches elektronisches Bauelement.
b. Das elektronische Bauteil ist ein lichtempfindlicher elektronischer Bildsensor zur Erfassung der elektromagnetischen Wellen.
c. Der mindestens eine lichtempfindliche elektronische Bildsensor ist ein CCD-Sensor oder ein CMOS-Sensor.

5. Implantat nach einem der vorherigen Ansprüche mit mindestens einem der folgenden zusätzlichen Merkmale:
a. Es umfasst mindestens eine Steuereinheit (106), die die Funktionen der Sensorik (104) und/oder der Datenübertragungseinheit (107) steuert.
b. Die Steuereinheit (106) ist dazu ausgebildet, eine unmittelbare Weiterleitung der von der Sensorik (104) gelieferten Daten mittels der Datenübertragungseinheit (107) an einen Empfänger zu veranlassen.
c. Die Steuereinheit (106) ist dazu ausgebildet, die von der Sensorik (104) gelieferten Daten zu speichern und zu einem späteren Zeitpunkt an die Datenübertragungseinheit (107) zu übertragen.
d. Die Steuereinheit (106) ist dazu ausgebildet, die von der Sensorik (104) gelieferten Daten zu bearbeiten, beispielsweise zu komprimieren, oder mit zuvor erfassten Daten oder gespeicherten Referenzdaten zu vergleichen und/oder zu kombinieren.
e. Die Steuereinheit (106) ist in dem Hohlraum (103) mit der Sensorik (104) oder in einem weiteren Hohlraum innerhalb des Implantats angeordnet.

6. Implantat nach einem der vorherigen Ansprüche mit mindestens einem der folgenden zusätzlichen Merkmale:
a. Es umfasst eine Lichtquelle (105), die Licht im für das menschliche Auge sichtbaren Bereich des elektromagnetischen Spektrums emittieren kann.
b. Es umfasst eine Strahlungsquelle, die elektromagnetische Strahlung außerhalb des sichtbaren Bereichs, insbesondere IR-Strahlung oder UV-Strahlung, emittieren kann.
c. die Licht- und/oder die Strahlungsquelle (105) ist mit der Steuereinheit (106) gekoppelt, die ihre Funktionen kontrolliert.
d. Die Licht- und/oder die Strahlungsquelle (105) ist in dem Hohlraum (103) mit der Sensorik (104), dem Hohlraum (103) mit der Steuereinheit (106) oder in einem weiteren Hohlraum innerhalb des Implantats angeordnet.

7. Implantat nach einem der vorherigen Ansprüche mit mindestens einem der folgenden zusätzlichen Merkmale:
a. Es umfasst mindestens eine Energiequelle (108), die die Sensorik (104) und die Datenübertragungseinheit (107) sowie gegebenenfalls die Steuereinheit (106) und/oder die Licht- und/oder die Strahlungsquelle (105) mit elektrischer Energie versorgt.
b. Die mindestens eine Energiequelle (108) umfasst eine elektrochemische Zelle.
c. Die mindestens eine Energiequelle umfasst einen Kondensator.
d. Die mindestens eine Energiequelle (108) umfasst eine Induktionsspule/Kopplungsspule.
e. Die mindestens eine Energiequelle (108) umfasst einen Energiewandler.
f. Die Energiequelle (108) ist in dem Hohlraum (103) mit der Sensorik (104), dem Hohlraum (103) mit der Steuereinheit (106), dem Hohlraum (103) mit der Licht- und/oder die Strahlungsquelle (105) oder in einem weiteren Hohlraum innerhalb des Implantats angeordnet.

8. Implantat nach Anspruch 7 mit dem folgenden zusätzlichen Merkmal:
a. Die Induktionsspule/Kopplungsspule ist Bestandteil der Datenübertragungseinheit.

9. Implantat nach einem der vorherigen Ansprüche mit mindestens einem der folgenden zusätzlichen Merkmale:
a. Es umfasst eine Diagnosesubstanz zum Nachweis von Entzündungen.
b. Die Diagnosesubstanz ist auf eine Außenseite des Implantats aufgebracht.
c. Es umfasst ein Depot mit der Diagnosesubstanz.

10. Implantat nach einem der vorherigen Ansprüche mit mindestens einem der folgenden zusätzlichen Merkmale:
a. Es umfasst einen Wirkstoff zur Behandlung von Entzündungen.
b. Der Wirkstoff ist auf eine Außenseite des Implantats aufgebracht.
c. Es umfasst ein Depot mit dem Wirkstoff.

11. Implantat nach einem der vorherigen Ansprüche mit einem der folgenden zusätzlichen Merkmale:
a. Das künstliche Gelenk ist ausgewählt aus der Gruppe mit Schultergelenk, Ellenbogengelenk, Handgelenk, Hüftgelenk, Kniegelenk und Sprunggelenk.

## Claims

1. Medical Implant (100; 300; 400) selected from the group consisting of implant comprising an artificial joint, pacemaker and implantable hearing aid, having the following features:
a. It comprises a sensor (104) for detecting electromagnetic waves; and
b. It comprises a data transmission unit (107) which can wirelessly transmit data supplied by the sensor (104) to a receiving unit,
wherein for detecting the electromagnetic waves the sensor comprises at least one electronic component which is designed for the detection of electromagnetic waves within the visible range of the electromagnetic spectrum and/or the sensor is designed for the detection of electromagnetic waves in range from 200 nm to 700 nm.

2. Medical Implant according to claim 1, having the following additional features:
a. It comprises at least one cavity (103; 303; 403) in which the sensor (104) and the data transmission unit (107) are arranged, and
b. it is designed in such a way that the electromagnetic waves can reach the sensor (104) located in the cavity (103).

3. Implant according to claim 1, having one of the following additional features:
a. It consists at least in part of a glass, in particular of a transparent glass.
b. It consists at least in part of a glass ceramic.
c. It consists at least in part of a ceramic.
d. It consists at least in part of a plastics material, for example a polyolefin such as polyethylene.

4. Implant according to claim 1, having one of the following additional features:
a. The at least one electronic component which is comprised by the sensor (104) comprises a photoresistor, a photodiode, a photo transistor or other photosensitive electronic component.
b. The electronic component is a photosensitive electronic image sensor designed to detect electromagnetic waves.
c. The at least one photosensitive electronic image sensor is a CCD sensor or a CMOS sensor.

5. Implant according to claim 1, having one of the following additional features:
a. It comprises at least one control unit (106) which controls the functions of the sensor (104) and/or the data transmission unit (107).
b. The control unit (106) is designed to cause the data supplied by the sensor (104) to be forwarded directly to a receiving unit via the data transmission unit (107).
c. The control unit (106) is designed to store the data supplied by the sensor (104) and transmit them to the data transmission unit (107) at a later time.
d. The control unit (106) is designed to process, for example compress, compare and/or combine, the data supplied by the sensor (104) with previously collected data or stored reference data.
e. The control unit (106) is located in the cavity (103) containing the sensor (104) or in another cavity within the implant.

6. Implant according to claim 1, having one of the following additional features:
a. It comprises a light source (105) capable of emitting light in the range of the electromagnetic spectrum visible to the human eye.
b. It comprises a radiation source capable of emitting electromagnetic radiation outside the visible range, in particular IR radiation or UV radiation.
c. The light and/or radiation source (105) is coupled to the control unit (106) which controls its functions.
d. The light and/or radiation source (105) is located in the cavity (103) containing the sensor (104), the cavity (103) containing the control unit (106) or in another cavity within the implant.

7. Implant according to claim 1, having one of the following additional features:
a. It comprises at least one energy source (108) supplying electrical energy to the sensor (104) and the data transmission unit (107) and, where appropriate, to the control unit (106) and/or the light and/or radiation source (105).
b. The at least one energy source (108) comprises an electrochemical cell.
c. The at least one energy source comprises a capacitor.
d. The at least one energy source (108) comprises an induction coil or coupling coil.
e. The at least one energy source comprises an energy converter.
f. The energy source (108) is located in the cavity (103) containing the sensor (104), the cavity (103) containing the control unit (106), the cavity (103) containing the light and/or radiation source (105) or in another cavity within the implant.

8. Implant according to claim 7, having one of the following additional feature:
a. The induction coil/coupling coil is part of the data transmission unit.

9. Implant according to claim 1, having one of the following additional features:
a. It comprises a diagnostic substance for the detection of inflammation.
b. The diagnostic substance is positioned on an outer side of the implant.
c. It comprises a depot containing the diagnostic substance.

10. Implant according to claim 1, having one of the following additional features:
a. It comprises an active substance for the treatment of inflammation.
b. The active substance is applied on an outer side of the implant.
c. It comprises a depot containing the active substance.

11. Implant according to claim 1, having one of the following additional features:
a. The artificial joint is selected from the group consisting of the shoulder joint, elbow joint, wristjoint, hip joint, knee joint and ankle joint.

## Revendications

1. Implant (100 ; 300 ; 400) issu du groupe comprenant un implant avec une articulation artificielle, un stimulateur cardiaque et un appareil auditif implantable, ayant les particularités suivantes :
a. il comprend un système de capteurs (104) pour détecter des ondes électromagnétiques qui sont émises ou réfléchies par un environnement de l'implant, et
b. il comprend une unité de transmission de données (107) qui peut transmettre sans contact à un récepteur les données fournies par le système de capteurs (104),
dans lequel le système de capteurs comprend pour la détection des ondes électromagnétiques un composant électronique qui est conçu pour la détection d'ondes électromagnétiques dans le domaine visible du spectre électromagnétique, et/ou le système de capteurs est réalisé pour recevoir des ondes électromagnétiques dans la plage de 200 nm à 700 nm.

2. Implant selon la revendication 1 ayant les particularités supplémentaires suivantes :
a. il comprend au moins une cavité (103 ; 303 ; 403) dans laquelle sont disposés le système de capteurs (104) et l'unité de transmission de données (107), et
b. il est réalisé de telle sorte que les ondes électromagnétiques peuvent atteindre le système de capteurs (104) disposé dans la cavité (103).

3. Implant selon la revendication 1 ayant l'une des particularités supplémentaires suivantes :
a. Il est composé au moins partiellement de verre.
b. Il est composé au moins partiellement de vitrocéramique.
c. Il est composé au moins partiellement de céramique.
d. Il est composé au moins partiellement de matière plastique, en particulier de polyoléfine, comme le polyéthylène.

4. Implant selon l'une quelconque des revendications précédentes ayant au moins l'une des particularités supplémentaires suivantes :
a. Ledit au moins un composant électronique compris dans le système de capteurs (104) comprend une photorésistance, une photodiode, un phototransistor ou un autre composant électronique photosensible.
b. Le composant électronique est un capteur d'image électronique photosensible pour détecter les ondes électromagnétiques.
c. Ledit au moins un capteur d'image électronique photosensible est un capteur CCD ou un capteur CMOS.

5. Implant selon l'une quelconque des revendications précédentes ayant au moins l'une des particularités supplémentaires suivantes :
a. Il comprend au moins une unité de commande (106) qui commande les fonctions du système de capteurs (104) et/ou de l'unité de transmission de données (107) .
b. L'unité de commande (106) est réalisée pour provoquer une retransmission directe des données fournies par le système de capteurs (104) au moyen de l'unité de transmission de données (107) à un récepteur.
c. L'unité de commande (106) est réalisée pour stocker les données fournies par le système de capteurs (104) et pour les transmettre ultérieurement à l'unité de transmission de données (107).
d. L'unité de commande (106) est réalisée pour traiter les données fournies par le système de capteurs (104), par exemple pour les comprimer ou les comparer et/ou les combiner avec des données détectées précédemment ou avec des données de références stockées.
e. L'unité de commande (106) est disposée dans la cavité (103) avec le système de capteurs (104) ou dans une autre cavité à l'intérieur de l'implant.

6. Implant selon l'une quelconque des revendications précédentes ayant au moins l'une des particularités supplémentaires suivantes :
a. Il comprend une source de lumière (105) qui peut émettre de la lumière dans le domaine du spectre électromagnétique, visible pour l'œil humain.
b. Il comprend une source de rayonnement qui peut émettre le rayonnement électromagnétique en dehors du domaine visible, en particulier un rayonnement IR ou un rayonnement UV.
c. La source de lumière et/ou de rayonnement (105) est couplée à l'unité de commande (106) qui contrôle ses fonctions.
d. La source de lumière et/ou de rayonnement (105) est disposée dans la cavité (103) contenant le système de capteurs (104), dans la cavité (103) contenant l'unité de commande (106) ou dans une autre cavité à l'intérieur de l'implant.

7. Implant selon l'une quelconque des revendications précédentes ayant au moins l'une des particularités supplémentaires suivantes :
a. Il comprend au moins une source d'énergie (108) qui alimente en énergie électrique le système de capteurs (104) et l'unité de transmission de données (107), ainsi que le cas échéant l'unité de commande (106) et/ou la source de lumière et/ou de rayonnement (105) .
b. Ladite au moins une source d'énergie (108) comprend une cellule électrochimique.
c. Ladite au moins une source d'énergie comprend un condensateur.
d. Ladite au moins une source d'énergie (108) comprend une bobine d'inductance/ bobine de couplage.
e. Ladite au moins une source d'énergie (108) comprend un convertisseur d'énergie.
f. La source d'énergie (108) est disposée dans la cavité (103) contenant le système de capteurs (104), dans la cavité (103) contenant l'unité de commande (106), dans la cavité (103) contenant la source de lumière et/ou de rayonnement (105) ou dans une autre cavité à l'intérieur de l'implant.

8. Implant selon la revendication 7 ayant la particularité supplémentaire suivante :
a. La bobine d'inductance/ bobine de couplage fait partie de l'unité de transmission de données.

9. Implant selon l'une quelconque des revendications précédentes ayant au moins l'une des particularités supplémentaires suivantes :
a. Il comprend une substance de diagnostic pour déceler des inflammations.
b. La substance de diagnostic est appliquée sur une face extérieure de l'implant.
c. Il comprend un dépôt avec la substance de diagnostic.

10. Implant selon l'une quelconque des revendications précédentes ayant au moins l'une des particularités supplémentaires suivantes :
a. Il comprend une substance active pour traiter des inflammations.
b. La substance active est appliquée sur une face extérieure de l'implant.
c. Il comprend un dépôt avec la substance active.

11. Implant selon l'une quelconque des revendications précédentes ayant l'une des particularités supplémentaire suivantes :
a. L'articulation artificielle est sélectionnée dans le groupe composé de l'articulation de l'épaule, de l'articulation du coude, du poignet, de l'articulation de la hanche, de l'articulation du genou, et de la cheville.
